# EUROPEAN PATENT APPLICATION

(11) **EP 4 819 147 A1**
(43) Date of publication of application: **07.10.2026**
(21) Application number: 23960151.1
(22) Date of filing: 29.11.2023
(51) Int. Cl.: G16B 30/00

(54) **INFORMATION PROCESSING PROGRAM, INFORMATION PROCESSING METHOD, AND INFORMATION PROCESSING DEVICE**

(71) Applicant: FUJITSU LIMITED, Kawasaki-shi, Kanagawa 211-8588 (JP)
(72) Inventor: KURIBAYASHI, Sotaro, Kawasaki-shi, Kanagawa 211-8588 (JP); KATOH, Takashi, Kawasaki-shi, Kanagawa 211-8588 (JP); NAKAGAWA, Akira, Kawasaki-shi, Kanagawa 211-8588 (JP)
(74) Representative: Hoffmann Eitle
(86) International application number: PCT/JP2023/042757
(87) International publication number: WO 2025/115131

(57) **Abstract**

An information processing program causes a computer to execute a process including: determining a weight of an input feature in a regression model, the regression model predicting an amino-acid sequence of a virus after mutation using an amino-acid sequence of the virus as the input feature, the determining being based on a first feature related to a three-dimensional (3D) structure of a protein of the virus and a second feature related to a contribution to prediction of a machine learning model, the contribution being obtained based on the first feature.

## Description

### [Technical Field]

The present embodiment is related to an information processing program, an information processing method, and an information processing device.

### [Background Art]

Since a virus frequently mutates, prediction of mutation is an important issue in developing vaccines against virus such as coronavirus.

Some conventional methods have predicted the amino-acid sequence after mutation by means of time-series analysis that regards a protein of a virus as an amino-acid sequence and associates the protein with the time of the epidemic, or LSTM (Long Short-Term Memory).

### [Prior Art Reference]

### [Patent Document]

[Patent Literature 1] International Publication Pamphlet No. 2022/019331
[Patent Literature 2] Japanese National Publication of International Patent Application No. 2022-521686
[Patent Literature 3] U.S. Patent Application Publication No. 2012/0265513
[Patent Literature 4] Japanese National Publication of International Patent Application No. 2022-527381
[Patent Literature 5] U.S. Patent Application Publication No. 2019/0266493

### [Summary of Invention]

### [Technical Problem]

However, such conventional methods for predicting virus mutation have difficulty in reflecting the influences between amino acids structurally distant from each other and the difference in the properties of the same amino acid located different positions in the virus when predicting the virus mutation.

Even if having the same chemical formula, some compounds, such as isomers, have different property and formation. Such conventional methods for predicting virus mutation have difficulty in following the compounds. Therefore, the accuracy in predicting virus mutation may be degraded.

According to an aspect, one of the objects of the present disclosure is to enhance the accuracy in predicting virus mutation.

### [Solution to Problem]

For this purpose, an information processing program causes a computer to execute a process including: determining a weight of an input feature in a regression model, the regression model predicting an amino-acid sequence of a virus after mutation using an amino-acid sequence of the virus as the input feature, the determining being based on a first feature related to a three-dimensional (3D) structure of a protein of the virus and a second feature related to a contribution to prediction of a machine learning model, the contribution being obtained based on the first feature.

### [Advantageous Effects of Invention]

According to the embodiment, the accuracy in predicting mutation of a virus can be enhanced.

### [Brief Description of Drawings]

[FIG. 1]

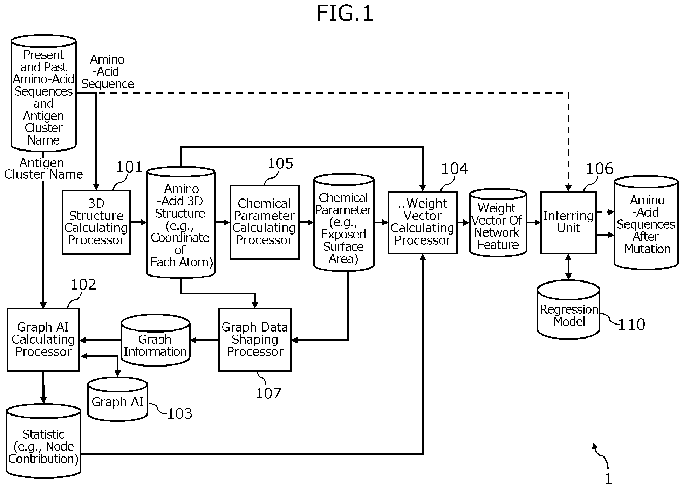

   FIG. 1 is a schematic diagram illustrating a configuration of an information processing device according to one embodiment.
[FIG. 2]

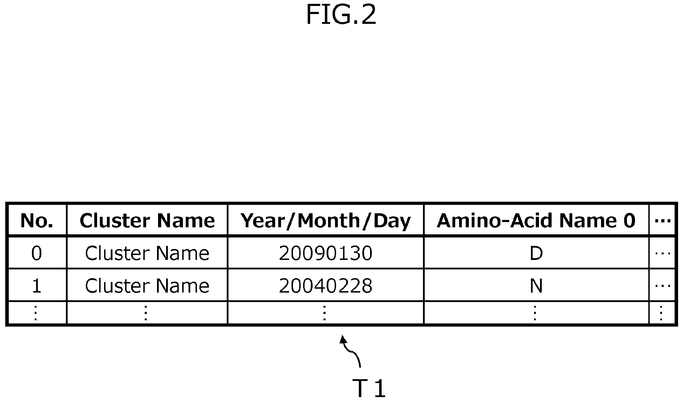

   FIG. 2 is a diagram illustrating an amino-acid sequence and an antigen cluster name used in the information processing device of the one embodiment.
[FIG. 3]

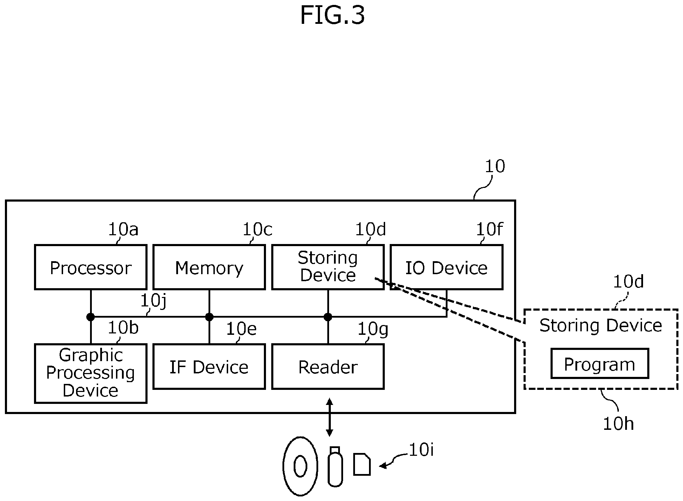

   FIG. 3 is a block diagram illustrating an example of a hardware configuration of a computer that achieves a function of the information processing device of the one embodiment.
[FIG. 4]

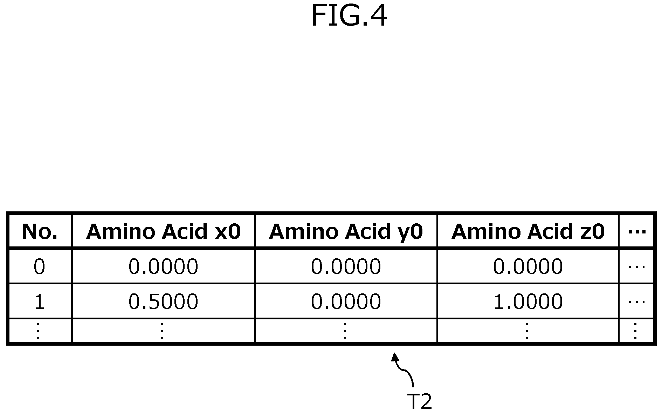

   FIG. 4 is a diagram showing amino-acid 3D structure information output from a 3D structure calculating processor in the information processing device of the one embodiment.
[FIG. 5]

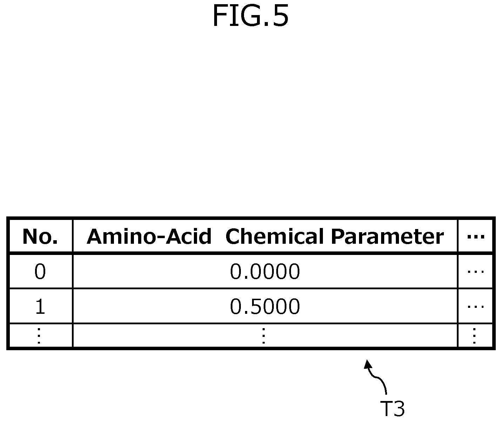

   FIG. 5 is a diagram illustrating chemical parameter information that a chemical parameter calculating processor generates in the information processing device of the one embodiment.
[FIG. 6]

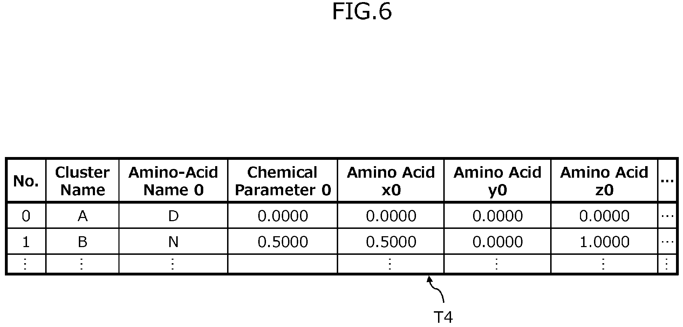

   FIG. 6 is a diagram illustrating graph information in the information processing device of the one embodiment.
[FIG. 7]

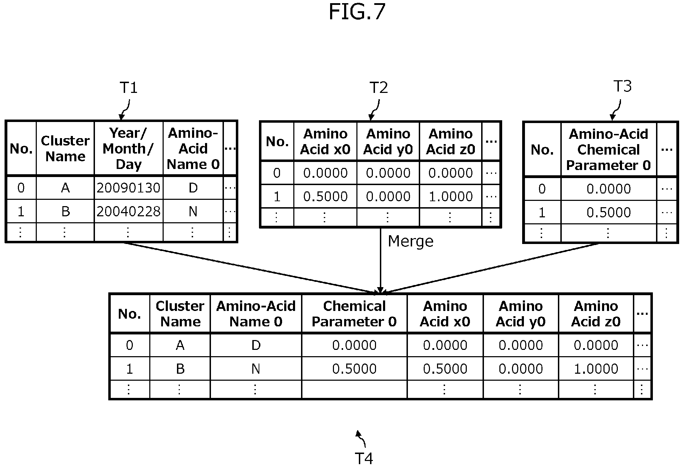

   FIG. 7 is a diagram illustrating a process that a graph data shaping processor carries out in the information processing device of the one embodiment.
[FIG. 8]

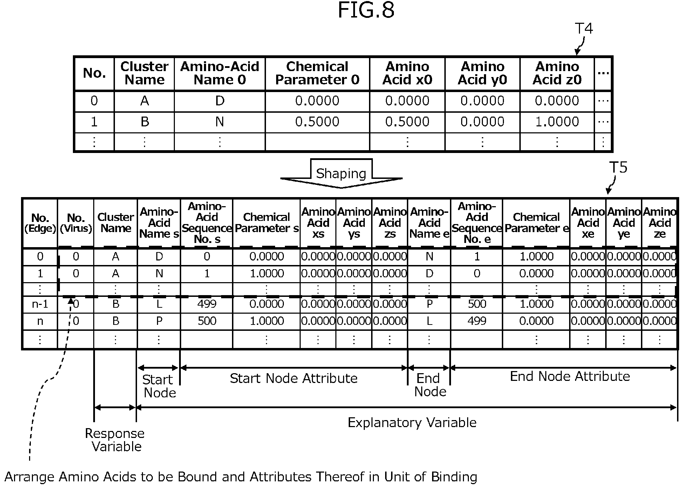

   FIG. 8 is a diagram illustrating graph AI input information in the information processing device of the one embodiment.
[FIG. 9]

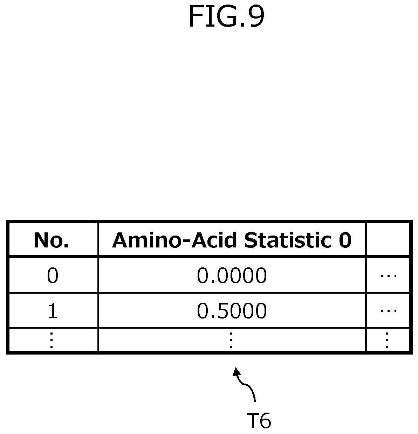

   FIG. 9 is a diagram illustrating statistical information in the information processing device of the one embodiment.
[FIG. 10]

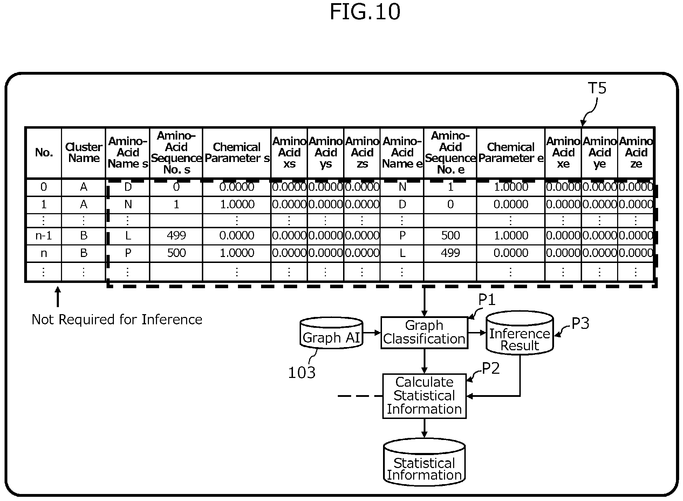

   FIG. 10 is a diagram illustrating a process that a graph AI calculating processor carries out in the information processing device of the one embodiment.
[FIG. 11]

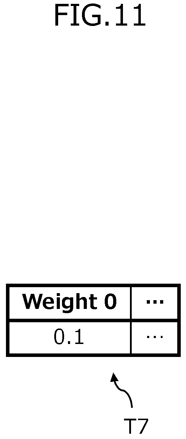

   FIG. 11 is a diagram illustrating an example of a weight vector in the information processing device of the one embodiment.
[FIG. 12]

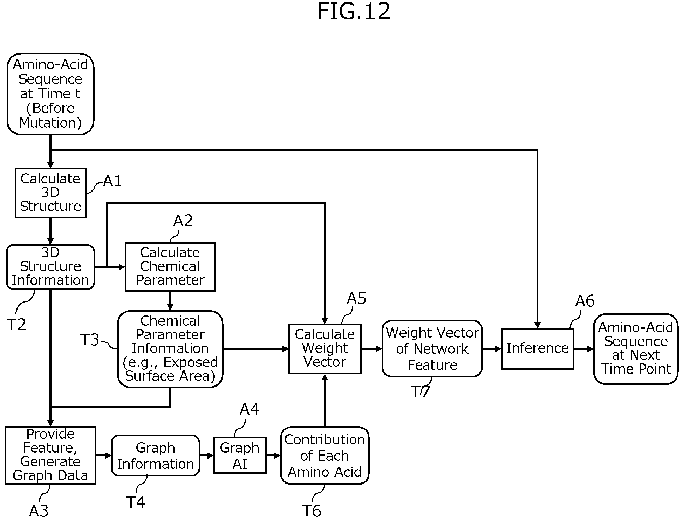

   FIG. 12 is a flow chart illustrating a process of a training phase in the information processing device of the one embodiment.
[FIG. 13]

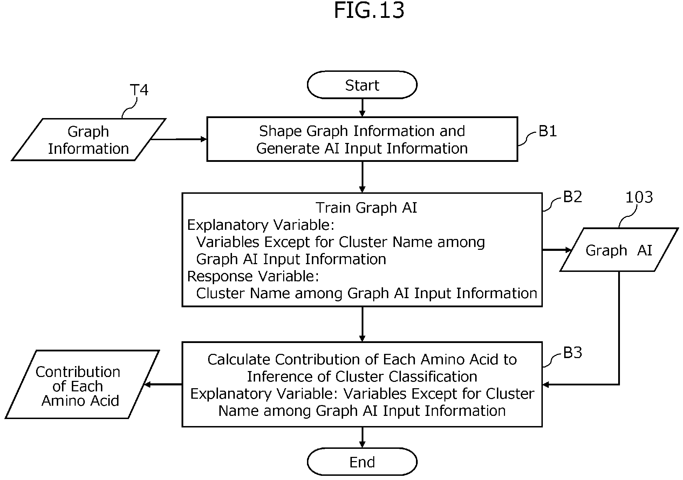

   FIG. 13 is a flow chart illustrating a process that the graph AI calculating processor carries out in the information processing device of the one embodiment.

### [Description of Embodiments]

Hereinafter, description will now be made in relation to a program for processing information, a method for processing information, and an information processing device according to the present embodiment with reference to the accompanying drawings. However, the following embodiment is merely illustrative and is not intended to exclude the application of various modifications and techniques not explicitly described in the embodiment. Namely, the present embodiment can be variously modified and implemented without departing from the scope thereof. Further, each of the drawings can include additional functions not illustrated therein to the elements illustrated in the drawing.

### (A) Configuration:

FIG. 1 is a diagram schematically illustrating a configuration of an information processing device 1 according to one embodiment.

The present information processing device 1 performs training (machine-learning) of a regression model 110 that predicts an amino-acid sequence of a protein of a virus after mutation (training phase).

In the training phase, an amino-acid sequence and an antigen cluster name of a virus at a certain past time point are input into the information processing device 1, and the amino-acid sequence of the virus (i.e., mutated virus) after the mutation are used as ground truth data.

Such a virus at a certain past time point may be simply referred to as a "past virus". In addition, an amino acid contained in this past virus may be referred to as a "past amino acid". An antigen cluster name may be simply referred to as a "cluster name". In addition, an amino-acid sequence and an antigen cluster of a past virus may be referred to as a "past amino-acid sequence" and a "past antigen cluster name".

In addition, in the present information processing device 1, an inferring unit 106 uses the trained regression model 110 for prediction (inference) of an amino-acid sequence of the protein of a mutated virus (predicting phase).

In predicting phase, an amino-acid sequence of a current (latest) virus is input into the information processing device 1, and the regression model 110 predicts the amino-acid sequence of the same virus (i.e., mutated virus) after the mutation. In the predicting phase, such an amino-acid sequence of the same virus after the mutation that the regression model 110 predicts on the basis of the input amino-acid sequence and the input antigen cluster name of the current (latest) virus may be referred to as a future amino-acid sequence.

FIG. 2 is a diagram illustrating an amino-acid sequence and an antigen cluster name information used in the information processing device 1 according to the one embodiment.

In FIG. 2, the amino-acid sequence and antigen cluster name information is represented in a data table format. Hereinafter, the amino-acid sequence and antigen cluster name information is sometimes represented by attaching thereto the reference sign T1.

The amino-acid sequence and antigen cluster name information T1 illustrated in FIG. 2 associates No., cluster name, year/month/day, and an amino-acid names with one another.

In the amino-acid sequence and antigen cluster name information T1 illustrated in FIG. 2, each of the data pieces represented by a letter string for convenience may be practically an integer value uniquely associated with the data piece. Data expressed in an integral value can be used efficiently for various computations and is highly convenient. The same is applied to various types of information to be described below.

The field of "No." represents information for identifying a virus. The field of "cluster name" represents an antigen cluster name of the virus. The field of "year/month/day" may represent the date and time when the virus appeared or was discovered. The amino-acid name indicates the type of amino acid contained in the virus, and represents any one of the 20 types of amino acids. In FIG. 2, for convenience, the amino-acid names (amino acid types) are represented using the letters, such as D and N.

If a virus includes multiple amino acids, the amino-acid sequence and antigen cluster name information T1 may list the multiple amino-acid names in association with the virus. The order of the amino-acid names may be, for example, arranged from the beginning to the end in the order of the peptide bond.

The multiple amino acids contained in a virus may be represented by numbers. A number representing an amino acid contained in a virus may be referred to as an amino-acid number. In the example illustrated in FIG. 2, the amino-acid number 0, which attaches an amino-acid number "0" to an amino-acid name, represents the 0-th amino acid among multiple amino acids included in a virus.

The amino-acid sequence and antigen cluster name information T1 may be prepared by a user, for example. In addition, for example, a non-illustrated processor may generate the amino-acid sequence and antigen cluster name information T1 by extracting information of an amino acid and an antigen cluster name from information of a known virus.

### (A-1) Example of Hardware Configuration:

The function of the information processing device 1 of the one embodiment may be achieved by one computer or by two or more computers. Further, at least a part of the functions of the information processing device 1 may be implemented using Hardware (HW) resources and Network (NW) resources provided by cloud environment.

FIG. 3 is a block diagram illustrating an example of a hardware (HW) configuration of the computer 10 that achieves the function of information processing device 1 according to the one embodiment. If multiple computers are used as the HW resources for achieving the functions of the information processing device 1, each of the computers may include the HW configuration illustrated in FIG. 3.

As illustrated in FIG. 3, the computer10 may illustratively include, as the HW configuration, a processor 10a, a graphic processing device 100b, a memory 10c, a storing device 10d, an Interface (IF) device 10e, an Input/Output (IO) device 10f, and a reader 10g.

The processor 10a is an example of an arithmetic processing device that performs various types of control and calculations and serving as a controller that carries out various processes. The processor 10a may be mutually communicably connected to each of the blocks in the computer 10 via a bus 10j. The processor 10a may be a multiprocessor including multiple processors or a multi-core processor including multiple processor cores, or may have a structure including two or more multi-core processors.

The processor 10a may be any one of integrated circuits (ICs) such as CPUs (Central Processing Units), MPUs (Micro Processing Units), APUs (Accelerated Processing Units), DSPs (Digital Signal Processors), ASICs (Application Specific Integrated Circuits), and FPGAs (Field Programmable Gate Arrays), or combinations of two or more of these ICs.

The graphic processing device 10b carries out screen displaying control on an output device such as a monitor serving as one of the IO device 10f. The graphic processing device 10b may have a function as an accelerator that executes a machine learning process and a predicting process using a machine learning model. Examples of the graphic processing device 10b are various ICs such as Graphic Processing Units (GPUs), APUs, DSPs, ASICs and FGPAs.

The memory 10c is an example of a hardware device that stores various pieces of data and information of a program. Examples of the memory 10c are one of a volatile memory such as a Dynamic Random Access Memory (DRAM) and a non-volatile memory such as a persistent Memory (PM) or the both.

The storing device 10d is an example of a hardware device that stores information such as various data, programs, and the like. Examples of the storing device 10d may be various storing devices including a magnetic disk device such as a Hard Disk Drive (HDD), a semiconductor drive device such as a Solid State Drive (SSD), a nonvolatile memory, and the like. The non-volatile memory may be, for example, a flash memory, a Storage Class Memory (SCM), a Read Only Memory (ROM), and the like.

The storing device 10d may store a program 10h (information processing program) that implements all or a part of various functions of the computer 10.

For example, the processor 10a of the information processing device 1 may achieve the function in a training phase and the function in a predicting phase to be detailed below by expanding the program 10h stored in the storing device 10d onto the memory 10c and executing the expanded program 10h.

The IF device 10e is an example of a communication IF that controls connections and communications between the computer 10 and other computer. For example, the IF device 10e may include an applying adapter conforming to Local Area Network (LAN) such as Ethernet^{®} or optical communication such as Fibre Channel (FC). The applying adapter may be compatible with either or both of wireless and wired communication schemes.

For example, the computer 10 may be communicably connected to a non-illustrated another computer and a database via the IF device 10e and a network. Furthermore, the program 10h may be downloaded from the network to the computer 10 through the communication IF device 10e and be stored in the storing device 10d.

The IO device 10f may include one or both of an input device and an output device. Examples of the input device include a keyboard, a mouse, and a touch panel. Examples of the output device include a monitor, a projector, and a printer. The IO device 10f may include, for example, a touch panel that integrates an input device and an output device with each other. The output device may be connected to the graphic processing device 10b.

The reader 10g is an example of a reader that reads information of data and programs recorded on a recording medium 10i. The reader 10g may include a connecting terminal or device to which the recording medium 10i may be connected or inserted. Examples of the reader 10g include an applying adapter conforming to, for example, Universal Serial Bus (USB), a drive apparatus that accesses a recording disk, and a card reader that accesses a flash memory such as an SD card. The program 10h may be stored in the recording medium 10i. The reader 10g may read the program 10h from the recording medium 10i and store the read program 10h into the storing device 10d.

Examples of the recording medium 10i illustratively include a non-transitory computer-readable recording medium such as a magnetic/optical disk, and a flash memory. Examples of the magnetic/optical disk include a flexible disk, a Compact Disc (CD), a Digital Versatile Disc (DVD), a Blu-ray disk, and a Holographic Versatile Disc (HVD). Examples of the flash memory include a semiconductor memory such as a USB memory and an SD card.

The HW configuration of the computer 10 described above is exemplary. Accordingly, the computer 10 may appropriately undergo increase or decrease of HW devices (e.g., addition or deletion of arbitrary blocks), division, integration in an arbitrary combination, or addition or deletion of the bus.

### (A-2) Examples of Functional Configuration:

As illustrated in FIG. 1, the information processing device 11 (sic, correctly, "1") may exemplarily have functions as a 3D structure calculating processor 101 a graph AI calculating processor 102, a graph AI 103, a weight vector calculating processor 104, a chemical parameter calculating processor 105, an inferring unit 106, a graph data shaping processor 107, and a regression model 110. These functions may be implemented by the hardware of a computer 10 (see FIG. 3). The term "AI" is an abbreviation for Artificial Intelligence.

The 3D structure calculating processor 101 analyzes the three-dimensional (3D) structure of the protein of a virus. When an amino-acid sequence of a virus is input, the 3D structure calculating processor 101 analyzes the three-dimensional structure of the amino acid (protein). The 3D structure calculating processor 101 outputs the 3D structure information of the amino acid as a result of the analysis. The 3D structure information of an amino acid may include, for example, a coordinate of each atom.

The function of the 3D structure calculating processor 101 may be realized by using a known structure calculating tool for a protein. For example, AlphaFold2 may be used as a structure calculating tool for a protein.

FIG. 4 is a diagram illustrating amino-acid 3D structure information that the 3D structure calculating processor 101 outputs in the information processing device 1 according to the one embodiment.

In FIG. 4, the amino-acid 3D structure information is represented in a data table format. Hereinafter, the amino-acid 3D structure information is sometimes represented by attaching thereto the reference sign T2.

In the amino-acid 3D structure information T2 illustrated in FIG. 4, a coordinate value of each amino acid is associated with the "No." that specifies a virus.

The coordinate value of each amino acid includes x, y, and z coordinate values. In FIG. 4, the coordinate of the amino acid having the amino-acid umber 0 is represented by attaching an amino-acid number 0 to each of the amino acid x, the amino acid y, and the amino acid z, for example.

Also in the amino-acid 3D structure information T2, the order of the amino-acid names may be, for example, from the beginning to the end in the order of the peptide bond.

The amino-acid 3D structure information T2 corresponds to a first feature related to a 3D structure of the protein of a virus. The amino-acid 3D structure information T2 that the 3D structure calculating processor 101 outputs may be stored in, for example, a predetermined storing region of the memory 10c or the storing device 10d.

The chemical parameter calculating processor 105 generates a chemical parameter for each amino acid included in a virus on the basis of the amino-acid 3D structure information that the 3D structure calculating processor 101 generates. An example of the chemical parameter may be charge. The chemical parameter calculating processor 105 may calculate charges for each amino acid, for example.

The chemical parameter calculating processor 105 may generate the chemical parameter, using various known techniques. For example, the chemical parameter calculating processor 105 may calculate features, such as charge, by using a known molecular dynamics simulator.

FIG. 5 is a diagram illustrating chemical parameter information generated by the chemical parameter calculating processor 105 in the information processing device 1 according to the one embodiment.

In FIG. 5, this chemical parameter information is represented in a data table format including multiple chemical parameters. Hereinafter, the chemical parameter information is sometimes represented by attaching thereto the reference sign T3.

The chemical parameter information T3 illustrated in FIG. 5 associates values of a chemical parameter of multiple amino acids with a "No." that specifies a virus.

In FIG. 5, for example, the amino-acid chemical parameter 0, which attaches the amino-acid number 0 to the amino-acid chemical parameter, represents the chemical parameter of an amino-acid having the amino-acid number 0.

Also in the chemical parameter information T3, the order of the amino-acid names may be, for example, from the beginning to the end in the order of the peptide bond.

In addition, the chemical parameter calculating processor 105 may generate multiple types of chemical parameter for each amino acid.

The chemical parameter information T3 corresponds to the third feature related to a property caused by a 3D structure. The chemical parameter information T3 generated by the chemical parameter calculating processor 105 may be stored in a predetermined in a predetermined storing region of the memory 10c or the storing device 10d. The first feature related to the 3D structure of protein of a virus may include the third feature related to a property caused by the 3D structure.

The graph data shaping processor 107 generates graph information based on the amino-acid 3D structure information T2 generated by the 3D structure calculating processor 101 and the chemical parameter information T3 generated by the chemical parameter calculating processor 105. The graph information may be also referred to as graph data.

FIG. 6 is a diagram illustrating the graph information in the information processing device 1 according to the one embodiment.

In FIG. 6, the graph information is represented in a data table format. Hereinafter, the graph information is sometimes represented by attaching thereto the reference sign T4.

FIG. 7 is a diagram illustrating a process performed by the graph data shaping processor 107 in the information processing device 1 according to the one embodiment.

The graph data shaping processor 107 generates a graph information T4 by merging (combining) the amino-acid sequence and antigen cluster name information T1, the amino-acid 3D structure information T2, and the chemical parameter information T3.

In generating the graph information T4, the graph data shaping processor 107 may merge the amino-acid sequence and antigen cluster name information T1, the amino-acid 3D structure information T2, and the chemical parameter information T3 on the basis of "No.", which specifies a virus.

The graph AI calculating processor 102 creates (shapes), based on the graph information T4 generated by the graph data shaping processor 107, data (graph AI input information T5) to be input into the graph AI 103.

The graph AI calculating processor 102 generates the graph AI input information T5 by converting information about multiple viruses included in the graph information T4 into data in the formats that the graph AI 103 can process.

The graph AI calculating processor 102 trains (machine learning) the graph AI 103 using the graph AI input information T5 in the training phase.

Here, the graph AI 103 is a machine learning model that performs graph-based relational learning, and achieves graph classification (class classification).

A graph is configured to include an aggregation of nodes and an aggregation of edges between the above nodes. It can be said that the graph is a mathematical model characterized by the nodes and the edges.

When the graph is applied to a virus, the amino acids correspond to the nodes, and bindings between the amino acids correspond to the edges. Binding between the amino acids may be, for example, the peptide bond, or may alternatively be binding by electrostatic force, or others.

The graph AI 103 performs graph classification based on the information of these graphs and these edges. In this classification, the amino-acid 3D structure may be used as an explanatory variable, and the antigen cluster name may be used as a response variable.

The graph classification may carry out the classification on the basis of the parameter of each node and each edge serving as a node attribute and an edge attribute, respectively.

In order to cause the graph AI 103 to perform the graph classification, the edges have to be explicitly provided to the graph AI 103. For this purpose, the graph AI calculating processor 102 assumes that adjacent amino acids have an edge on the basis of the amino-acid sequence. Furthermore, amino acids within a certain distance under the influence of, for example, electrostatic force are assumed to have an edge.

The function of the graph AI 103 can be achieved by using a known scheme. For example, the function of the graph AI 103 may be achieved by Deep Tensor (registered trademark).

The graph AI 103 corresponds to machine learning model that uses the amino-acid 3D structure information T2 (first feature related to the 3D structure of a protein of a virus) and the chemical parameter information T3 (third feature related to the 3D structure caused by the 3D structure) as input data.

The graph AI calculating processor 102 once carries out class classification of antigen clusters of the viruses on the basis of the 3D structure by means of the graph AI 103, and calculates a contribution of each amino acid after the class classification. The class classification of antigen clusters of the viruses on the basis of the 3D structure by means of the graph AI 103 is an example of prediction with a machine learning model that uses the first features and the third features as input data.

On the basis of the graph information T4, the graph AI calculating processor 102 generates the graph AI input information T5 by arranging, for each edge in an amino-acid sequence forming a virus, the attributes of two amino acids that the edge binds to each other in a unit of binding. Hereinafter, the two amino acids that an edge binds to each other may be referred to as an amino-acid pair. The amino acid at the beginning of the edge of an amino-acid pair may be referred to as a starting node, and the amino acid at the end of the edge may be referred to as an end node.

FIG. 8 is a diagram illustrating the graph AI input information T5 in the information processing device 1 according to the one embodiment.

FIG. 8 illustrates the graph information T4 illustrated in FIG. 6 and the graph AI input information T5 that the graph AI calculating processor 102 generates on the basis of the graph information T4.

The graph AI input information T5 illustrated in FIG. 8 associates information of the amino-acid pair that an edge binds to each other with the "No.", which that specifies the edge.

The information of an amino-acid pair includes "No." that specifies virus, a cluster name, and amino-acid name, an amino-acid sequence number, a chemical parameter, and coordinate values (x, y, z) of at the starting node and the end node. In the example illustrated in FIG. 8, the symbol "s" is attached to the end of each piece of information of the starting node, and the symbol "e" is attached to the end of each piece of information of the end node.

Accordingly, for example, the amino-acid name s represents the starting node and the amino-acid name e represents the end node. In addition, the amino-acid sequence number s, the chemical parameter s, the amino acid xs, the amino-acid name ys, and the amino acid zs represent attribute information (starting node attribute) of the starting node. Similarly, the amino-acid sequence number e, the chemical parameter e, the amino acid xe, the amino-acid name ye and the amino acid ze represent attribute information (end node attribute) of the end node.

In the training phase, the graph AI calculating processor 102 trains the graph AI 103, using the graph AI input information T5 as training data.

In the graph AI input information T5 illustrated in FIG. 8, the cluster name is used as a response variable in the training phase of the graph AI 103. The amino-acid name s, the amino-acid name e, the starting node attributes, and the end node attributes are used as explanatory variables in the training phase of the graph AI 103.

The graph AI 103 may be a Deep Neural Network (DNN) that includes multiple hidden layers between an input layer and an output layer.

For example, a NN executes a process (forward propagation process) in the forward direction in which process the information obtained by the calculations is sequentially transmitted from the input side to the output side by inputting input data into an input layer and sequentially executing predetermined calculations in the hidden layers composed of a convolutional layer, a pooling layer, or the like. After executing the processing in the forward direction, the NN executes a process (backward propagation process) in the backward direction in which process a parameter to be used in the process in the forward direction is determined in order to reduce the value of an error function obtained from output data (result of the graph classification) output from the output layer and the ground truth data (cluster name). Then, an updating process that updates a variable such as a weight is executed based on the result of the backward propagation process. For example, a gradient descent method may be used as an algorithm to determine the updating width of a weight to be used in the calculation of the backward propagation process.

In addition, in the training phase, the graph AI calculating processor 102 inputs the graph AI input information T5 into the graph AI 103 and causes the graph AI 103 to carry out graph classification (class classification) and then calculate statistical information.

The statistical information may be, for example, a contribution (contribution score, node contribution) for obtaining a result of the prediction when the graph AI 103 performs the graph classification. The statistical information may be referred to as statistic. The graph AI calculating processor 102 obtains statistic for each amino acid contained in the virus. The graph AI calculating processor 102 generates statistic (node contribution) for each amino acid on the basis of the statistical information.

The statistic of each amino acid included in a virus corresponds to a second feature (statistical feature) based on a contribution (statistical information) of each amino acid included in the protein to the prediction. Accordingly, the graph AI calculating processor 102 obtains second features (statistical features) based on contribution (statistical information) each amino acid contained in the protein to the prediction of through the prediction using the graph AI 103.

FIG. 9 is a diagram illustrating the statistical information in the information processing device 1 according to the one embodiment.

In FIG. 9, multiple pieces of the statistical information are represented in a data table format. Hereinafter, the statistical information is sometimes represented by attaching thereto the reference sign T6.

The statistical information T6 illustrated in FIG. 8 associates values of the statistical information of multiple amino acids with the "No." that specifies a virus.

In FIG. 9, for example, the amino-acid statistical information 0, which attaches the amino-acid number 0 to the amino-acid statistical information, represents the amino-acid statistical information of an amino-acid having the amino-acid number 0.

Also in the statistical information T6, the order of the amino-acid names may be, for example, from the beginning to the end in the order of the peptide bond.

The statistical information that the graph AI calculating processor 102 generates may be stored in, for example, a predetermined storing region of the memory 10c or the storing device 10d.

In the graph AI (graph AI 103), the contribution is obtained for each 3D structure and for each amino acid. For the above, the graph AI calculating processor 102 may obtain a sample mean of contribution in predetermined units of, for example, a cluster, a year, and an amino acid, and may use the obtained sample mean as the statistical information.

The result of the prediction that the graph AI calculating processor 102 causes the graph AI 103 to carry out and the values of the statistical information that the graph AI calculating processor 102 causes the graph AI 103 to calculate may be stored in, for example, a predetermined storing region of the memory 10c or the storing device 10d.

FIG. 10 is a diagram illustrating a process of the graph AI calculating processor 102 of the information processing device 1 according to the one embodiment.

As described above, in the training phase, the graph AI calculating processor 102 inputs the graph AI input information T5 into the graph AI 103 and causes the graph AI 103 to perform the graph classification (see the reference sign P1). In addition, the graph AI calculating processor 102 obtains the statistical information (contribution) that the graph AI 103 calculates (see the reference sign P2).

The graph AI calculating processor 102 shifts the values included in the graph AI input information T5 and confirms how the result of the inference changes (see the reference sign P3). If the result of the inference improves, the graph AI calculating processor 102 may process the graph AI input information T5 to reflect the change.

Into the weight vector calculating processor 104, the 3D structure information (amino-acid 3D structure information T2) of the amino acid generated by the 3D structure calculating processor 101, the chemical parameter (chemical parameter information T3) generated by the chemical parameter calculating processor 105, and the statistic (node contribution: statistical information T6) for each amino acid generated by the graph AI calculating processor 102 are input.

The weight vector calculating processor 104 generates a fixed-length vector (weight of the input feature) for each amino-acid sequence, using these pieces of information. The weight vector of the feature is used as a weight of feature (input feature) input into a regression model 110 (NN: Neural Network) to be described below.

The weight vector calculating processor 104 determines, based on the amino-acid 3D structure information T2 (first features), the chemical parameter information T3 (third features), and the statistical features (second features), a weight (weight vector) of the input feature in the regression model 110.

For example, the weight vector calculating processor 104 may set a weight for an amino-acid sequence by embedding graph data in a fixed-length vector by using a function, such as a transformer, which is a known machine learning model. In other words, the weight vector calculating processor 104 sets a numeric value of the regularity, such as the importance, associated with the amino-acid sequence.

FIG. 11 is a diagram illustrating weight vector information in the information processing device 1 according to the one embodiment.

In FIG.11, multiple pieces of weight vector information are represented in a data table format. Hereinafter, the weight vector information is sometimes represented by attaching thereto the reference sign T7.

In FIG. 11, for example, the weight 0, which attaches the amino-acid number 0 to the weight, represents the weight of an amino-acid having the amino-acid number 0.

Also in the weight vector information T7, the order of the amino-acid names may be, for example, arranged from the beginning to the end in the order of the peptide bond.

The weight vector calculating processor 104 determines hyperparameters, such as dimensions of input/output variables and latent variables of a model on the basis of a contribution of each amino acid.

The weight vector information T7 is by no means limited to that illustrated in FIG. 11, and can be appropriately modified and implemented. Alternatively, multiple weight vectors may be provided for one virus. These multiple weight vectors may be managed in the chronological order.

The weight vector information that the weight vector calculating processor 104 generates may be stored in, for example, a predetermined storing region of the memory 10c or the storing device 10d.

The inferring unit 106 predicts (infers) an amino-acid sequence of a virus after mutation.

For example, the inferring unit 106 predicts amino-acid sequence of the virus after mutation, using the regression model 110.

The inferring unit 106 trains the regression model 110 in the training phase, and causes the regression model 110 to predict the amino-acid sequence after the mutation in the predicting phase.

The inferring unit 106 predicts an amino-acid sequence at the time t+Δt (Δt>0) on the basis of the amino-acid sequence at time t, using the regression model 110.

The regression model 110 may achieve regression by using a scheme such SVR, NN, GA (Genetic Algorithms), a time series analysis. In this regression, for example, an amino-acid sequence may be provisionally formed into a number sequence in association with a vector consisting of numbers, and a number sequence associated with an amino-acid name (e.g., 20 types such as proline) may be output in the form of a vector. For example, expressing an amino-acid sequence in numbers of 0 to 19, a regression problem as to which order these numbers are to be output may be solved.

Alternatively, the regression model 110 may be a deep neural network (DNN) that includes multiple hidden layers between the input-layer and the output-layer.

The regression model 110 corresponds to a regression model that predicts an amino-acid sequence of a virus after mutation using an amino-acid sequence of the virus as input features (explanatory variable).

In the training phase, the inferring unit 106 trains the regression model 110 that predicts an amino-acid sequence of a virus after mutation, using an amino-acid sequence of the virus and a weight vector (weight of the input features) of the features which vector the weight vector calculating processor 104 generates as the input features (explanatory variables).

The inferring unit 106 trains a machine learning model 100, using the amino-acid sequence and the weight vector of the features which weight vector is generated by the weight vector calculating processor 104 at the last time (time t) as the learning data and the amino-acid sequence at the ensuing time t+Δt (Δt>0) as the ground truth data.

As the above, by using the weight vector of features which weight vector is generated by the weight vector calculating processor 104 to train the regression model 110, the 3D structure of the protein of the virus can be reflected.

Here, the regression calculation assumes that the data lengths (dimension when vectorized) of the input and output are fixed. However, the lengths of the amino-acid sequences of viruses are not the same. In view of the above, amino-acid sequences having a fixed length may be generated and used by extracting a part of a predetermined length from amino-acid sequences. The generation of a fixed-length amino-acid sequence may extract, for example, a part of a predetermined length by excluding the leading part and the trailing part of an amino-acid sequence. The method of generating an amino-acid sequence of a fixed length is not limited to this, and can be appropriately modified and implemented.

In the predicting phase, only an amino-acid sequence is input into the inferring unit 106. The inferring unit 106 inputs the input amino-acid sequence into the regression model 110 and obtains the amino-acid sequence after mutation. In addition, the inferring unit 106 may also output statistic (e.g., contribution) that can be used to describe prediction.

### (B) Operation

Description will now be made in relation to a process of the training phase in the information processing device 1 of the one embodiment configured as the above with reference to a flow chart (Steps A1-A6) of FIG. 12.

Upon input of an amino-acid sequence of a virus at the time t into the 3D structure calculating processor 101, the 3D structure calculating processor 101 performs the three-dimensional structure analysis of the amino acid in Step A1. The 3D structure calculating processor 101 generates amino-acid 3D structure information T2.

The amino-acid 3D structure information T2 is input into the chemical parameter calculating processor 105. In Step A2, the chemical parameter calculating processor 105 generates a chemical parameter for each amino acid contained in the virus on the basis of the amino-acid 3D structure information T2, and generates chemical parameter information T3.

The amino-acid 3D structure information T2 generated by the 3D structure calculating processor 101 and the chemical parameter information T3 generated by the chemical parameter calculating processor 105 are input into the graph data shaping processor 107. In Step A3, the graph data shaping processor 107 generates graph information T4 based on the amino-acid 3D structure information T2 and the chemical parameter information T3.

The graph information T4 generated by the graph data shaping processor 107 is input into the graph AI 103. On the basis of the graph information T4, the graph AI calculating processor 102 generates graph AI input information T5 by arranging, for each edge in an amino-acid sequence forming a virus, the attributes of two amino acids that the edge binds to each other in a unit of binding.

The graph AI calculating processor 102 trains the graph AI 103, using the graph AI input information T5 as training data. In Step A4, the graph AI calculating processor 102 causes the graph AI 103 to calculate statistical information (contribution) and generates statistical information T6.

The contribution (statistical information T6) of each amino acid generated by the graph AI calculating processor 102 and the chemical parameter information T3 generated by chemical parameter calculating processor 105 are input into the weight vector calculating processor 104.

In Step A5, the weight vector calculating processor 104 generates a weight vector (weight vector information T7) of the feature of the NN using the contribution, the chemical parameter information T3, and the amino-acid 3D structure information T2 of each amino acid. This means that the weight vector calculating processor 104 determines hyperparameters, such as dimensions of input/output variables and latent variables of a model on the basis of a contribution of each amino acid.

The weight vector information T7 that the weight vector calculating processor 104 generates and the amino-acid sequence at the time t are input into the inferring unit 106.

In Step A6, the inferring unit 106 trains a machine learning model 100, using the amino-acid sequence and the weight vector of the features which weight vector is generated by the weight vector calculating processor 104 at the last time (time t) as the input features (explanatory variables) and the amino-acid sequence at the ensuing time t+Δt (Δt>0) as the ground truth data.

For example, the inferring unit 106 converts an amino-acid sequence into a fixed dimension, inputs the converted amino-acid sequence to the regression model 110, and causes the regression model 110 to predict the amino-acid sequence.

The inferring unit 106 compares the predicted amino-acid sequence with the ground truth data (amino-acid sequence after the mutation). With reference to the result of this comparison, the inferring unit 106 performs a process (backward propagation process) in the backward direction for determining a parameter to be used in a process in the forward direction in order to reduce the value of an error function to be obtained. The inferring unit 106 then performs the updating process that updates a variable such as a weight on the basis of the result of the backward propagation process.

In the predicting phase in the information processing device 1 of the one embodiment configured as the above, the present amino-acid sequence of the virus is input into the inferring unit 106. In step A6, the inferring unit 106 converts the amino-acid sequence into a fixed length, inputs the fixed-length amino-acid sequence into the regression model 110, causes the regression model 110 to predict an amino-acid sequence after mutation.

The amino-acid sequence that the regression model 110 outputs in the predicting phase may be used as training data in the subsequent training phase.

Next, description will now be made in relation to a process of the graph AI calculating processor 102 of the information processing device 1 according to the one embodiment having the above configuration with reference to a flow chart (Steps B1 to B3) illustrated in FIG. 13.

In Step B1, the graph AI calculating processor 102 shapes the graph information T4 generated by the graph data shaping processor 107 to generate graph AI input information T5.

In the training phase, the graph AI calculating processor 102 trains the graph AI 103, using the generated graph AI input information T5 (step B2).

At this time, the graph AI calculating processor 102 uses the graph AI input information T5 except for the cluster name as explanatory variables, and uses the cluster name as the response variable.

In Step B3, the graph AI calculating processor 102 inputs the graph AI input information T5 into the graph AI 103 and causes the graph AI 103 to predict (infer) the cluster name. At this time, the graph AI calculating processor 102 uses graph AI input information T5 except for the cluster name as the explanatory variables.

Then, the graph AI calculating processor 102 causes the graph AI 103 to calculate the statistical information (contribution of each amino acid). Then, the process ends.

### (C) Effect:

As the above, in the information processing device 1 according to the one embodiment, in the training phase that trains the regression model 110, which predicts the amino-acid sequence of a virus after mutation, the graph AI calculating processor 102 causes the graph AI 103 to carry out graph classification (prediction), using the amino-acid 3D structure information T2 (first feature) related to the 3D structure of the protein of the chemical parameter information T3 (third feature) related to a property originated from the 3D structure as inputs.

In addition, the graph AI calculating processor 102 inputs the graph AI input information T5 into the graph AI 103, and causes the graph AI 103 to perform graph classification (class classification) and then calculate the statistical information, so that the statistic (node contribution) of each amino acid is generated.

The weight vector calculating processor 104 generates a weight vector (weight of input feature) of each amino-acid sequence, using the amino-acid 3D structure information T2 that the 3D structure calculating processor 101 generates, the chemical parameter information T3 that the chemical parameter calculating processor 105 generates, and the statistic (node contribution) that the graph AI calculating processor 102 generates for each amino acid.

Then, the inferring unit 106 trains, in the training phase, the regression model 110 that predicts an amino-acid sequence of a virus after mutation, using the amino-acid sequence and the weight vector of the features which weight vector is generated by the weight vector calculating processor 104.

This reflects the 3D structure of the protein of the virus. Accordingly, since the regression model 110 can perform prediction of the virus mutation considering the property unique to the 3D structure of the protein of the virus in the predicting phase, the accuracy in the prediction can be enhanced.

A protein is composed of multiple amino acids bound via peptide bond, and an amino-acid sequence is a sequence of the amino acids in this order of the binding. However, amino acids distant from each other in an amino-acid sequence may be bound to each other via, for example, electrostatic force, which provides a unique shape and a unique property. This means that the same amino-acid sequence may have different features due to such a unique shape and a unique property.

In the present information processing device 1, the accuracy in the prediction can be enhanced by prediction the mutation of the virus from the feature based on the 3D structure of the protein.

### (D) Miscellaneous:

The disclosed techniques are not limited to the embodiment described above, and may be variously modified without departing from the scope of the present embodiment. The respective configurations and processes of the present embodiment can be selected, omitted, and combined according to the requirement.

For example, in the above-described embodiment, the chemical parameter information T3 is generated on the basis of the amino-acid 3D structure information T2. However, generation of the chemical parameter information T3 is not limited to this and alternatively does not have to be based on the amino-acid 3D structure information T2. Alternatively, the information processing device 1 does not have to generate the chemical parameter information T3, which may alternatively be obtained from an external entity.

For example, the above-described embodiment causes the graph AI 103 to perform the graph classification (prediction), using the amino-acid 3D structure information T2 (first features) and the chemical parameter information T3 (third features) as inputs. Alternatively, the graph AI 103 may be caused to perform the graph classification (prediction), using only the amino-acid 3D structure information T2 (first features) as the inputs.

For example, in the above-described embodiment, the graph AI calculating processor 102 generates the statistic (node contribution), and the weight vector calculating processor 104 generates a weight vector (weight of an input feature) of each amino-acid sequence, but alternatively the same processor may execute these processes.

For example, the above-described embodiment uses a contribution as the statistical information. The statistical information is not limited to this, and may alternatively be information except for the contribution.

The present embodiment can be executed or produced by those ordinary skilled in the art referring to the above disclosure.

### [Reference Signs List]

- 1: information processing device
- 10: computer
- 10a: processor
- 10b: graphic processing device
- 10c: memory
- 10d: storing device
- 10e: IF device
- 10f: IO device
- 10g: reader
- 10h: program
- 10i: recording medium
- 10j: bus
- 101: 3D structure calculating processor
- 102: graph AI calculating processor
- 103: graph AI
- 104: weight vector calculating processor
- 105: chemical parameter calculating processor
- 106: inferring unit 106
- 107: graph data shaping processor
- 110: regression model
- T1: amino-acid sequence and antigen cluster name information
- T2: amino-acid 3D structure information (first feature)
- T3: chemical parameter information (third feature)
- T4: graph information
- T5: graph AI input information
- T6: statistical information
- T7: weight vector information

## Claims

1. An information processing program that causes a computer to execute a process comprising:
determining a weight of an input feature in a regression model, the regression model predicting an amino-acid sequence of a virus after mutation using an amino-acid sequence of the virus as the input feature, the determining being based on a first feature related to a three-dimensional (3D) structure of a protein of the virus and a second feature related to a contribution to prediction of a machine learning model, the contribution being obtained based on the first feature.

2. The information processing program according to claim 1, wherein
the first feature includes a third feature related to a property caused by the 3D structure.

3. The information processing program according to claim 2, wherein the process further comprises:
obtaining the second feature based on the contribution of each amino acid included in the protein to the prediction associated with the first feature and the third feature being regarded as input data by prediction with the machine learning model using the input data; and
determining the weight of the input feature in the regression model that predicts an amino-acid sequence of the virus after the mutation by using the amino-acid sequence of the virus as the input feature, the determining being based on the first feature, the second feature, and the third feature.

4. The information processing program according to claim 3, wherein
a process of training the regression model uses the weight of the input feature and the amino-acid sequence of the virus as the input feature.

5. The information processing program according to claim 3 or 4, wherein the process further comprises
generating the first feature by analyzing the 3D structure of amino acids of the protein.

6. The information processing program according to claim 5, wherein the process further comprises
generating, based on the first feature, the third feature for each amino acid contained in the virus.

7. The information processing program according to claim 3, wherein
the weight of the input feature is a weight set for each amino-acid sequence of the virus.

8. A computer-implemented method for processing information comprising:
determining a weight of an input feature in a regression model, the regression model predicting an amino-acid sequence of a virus after mutation using an amino-acid sequence of the virus as the input feature, the determining being based on a first feature related to a three-dimensional (3D) structure of a protein of the virus and a second feature related to a contribution to prediction of a machine learning model, the contribution being obtained based on the first feature.

9. The computer-implemented method according to claim 8, wherein
the first feature includes a third feature related to a property caused by the 3D structure.

10. The computer-implemented method according to claim 9, further comprising:
obtaining the second feature based on the contribution of each amino acid included in the protein to the prediction associated with the first feature and the third feature being regarded as input data by prediction with the machine learning model using the input data; and
determining the weight of the input feature in the regression model that predicts an amino-acid sequence of the virus after the mutation by using the amino-acid sequence of the virus as the input feature, the determining being based on the first feature, the second feature, and the third feature.

11. The computer-implemented method according to claim 10, wherein
a process of training the regression model uses the weight of the input feature and the amino-acid sequence of the virus as the input feature.

12. The computer-implemented method according to claim 10 or 11, further comprising
generating the first feature by analyzing the 3D structure of amino acids of the protein.

13. The computer-implemented method according to claim 12, further comprising
generating, based on the first feature, the third feature for each amino acid contained in the virus.

14. The computer-implemented method according to claim 8, wherein
the weight of the input feature is a weight set for each amino-acid sequence of the virus.

15. An information processing device comprising a controller configured to execute a process comprising:
determining a weight of an input feature in a regression model, the regression model predicting an amino-acid sequence of a virus after mutation using an amino-acid sequence of the virus as the input feature, the determining being based on a first feature related to a three-dimensional (3D) structure of a protein of the virus and a second feature related to a contribution to prediction of a machine learning model, the contribution being obtained based on the first feature.

16. The information processing device according to claim 15, wherein
the first feature includes a third feature related to a property caused by the 3D structure.

17. The information processing device according to claim 16, wherein the process executed by the controller further comprises
obtaining the second feature based on the contribution of each amino acid included in the protein to the prediction associated with the first feature and the third feature being regarded as input data by prediction with the machine learning model using the input data; and
determining the weight of the input feature in the regression model that predicts an amino-acid sequence of the virus after the mutation by using the amino-acid sequence of the virus as the input feature, the determining being based on the first feature, the second feature, and the third feature.

18. The information processing device according to claim 17, wherein
a process of training the regression model uses the weight of the input feature and the amino-acid sequence of the virus as the input feature.

19. The information processing device according to claim 17 or 18, wherein the process executed by the controller further comprises
generating the first feature by analyzing the 3D structure of amino acids of the protein.

20. The information processing device according to claim 19, wherein the process executed by the controller further comprises
generating, based on the first feature, the third feature for each amino acid contained in the virus.

21. The information processing device according to claim 17, wherein
the weight of the input feature is a weight set for each amino-acid sequence of the virus.

22. An information processing program that causes a computer to execute a process comprising:
determining a weight of an input feature in a regression model, the regression model predicting an amino-acid sequence of a virus after mutation using an amino-acid sequence of the virus as the input feature, the determining being based on a first feature related to a three-dimensional (3D) structure of a protein of the virus and a second feature related to a contribution to prediction of a machine learning model, the contribution being obtained based on the first feature; and
training the regression model, using the weight of the input feature and the amino-acid sequence as input features.

23. An information processing device comprising a controller configured to execute a process comprising:
determining a weight of an input feature in a regression model, the regression model predicting an amino-acid sequence of a virus after mutation using an amino-acid sequence of the virus as the input feature, the determining being based on a first feature related to a three-dimensional (3D) structure of a protein of the virus and a second feature related to a contribution to prediction of a machine learning model, the contribution being obtained based on the first feature; and
training the regression model, using the weight of the input feature and the amino-acid sequence as input features.

24. An information processing device comprising a controller configured to execute a process comprising:
determining a weight of an input feature in a regression model, the regression model predicting an amino-acid sequence of a virus after mutation using an amino-acid sequence of the virus as the input feature, the determining being based on a first feature related to a three-dimensional (3D) structure of a protein of the virus and a second feature related to a contribution to prediction of a machine learning model, the contribution being obtained based on the first feature; and
training the regression model, using the weight of the input feature and the amino-acid sequence as input features.
